# EUROPEAN PATENT APPLICATION

(11) **EP 2 248 803 A1**
(43) Date of publication of application: **10.11.2010**
(21) Application number: 09714215.2
(22) Date of filing: 17.02.2009
(51) Int. Cl.: C07D 211/56, C07C 59/255, C07B 57/00

(54) **METHOD FOR OPTICAL RESOLUTION OF ALKYLPIPERIDIN-3-YL CARBAMATE AND INTERMEDIATE THEREFOR**

(30) Priority: 27.02.2008 JP 2008045847
(71) Applicant: Sumitomo Chemical Company, Limited, Tokyo 104-8260 (JP)
(72) Inventor: WATANABE, Yosuke, Toyonaka-shi Osaka 560-0021 (JP); YASUOKA, Junichi, Kobe-shi Hyogo 658-0073 (JP); IKEMOTO, Tetsuya, Toyonaka-shi Osaka 561-0874 (JP)
(74) Representative: Smyth, Gyles Darren
(86) International application number: PCT/JP2009/053125
(87) International publication number: WO 2009/107571

(57) **Abstract**

Disclosed is a method for optical resolution of an alkylpiperidin-3-yl carbamate, the method including bringing an RS mixture of an alkylpiperidin-3-yl carbamate into contact with an optically active tartaric acid in the presence of a solvent.

## Description

### TECHNICAL FIELD

The present invention relates to a method for optical resolution of an alkylpiperidin-3-yl carbamate, and an intermediate therefor.

### BACKGROUND ART

As a method of producing an optically active 3-aminopiperidine, for example, a method by optical resolution of 3-aminopiperidine is known (see Patent Document 1). However, such a method was not industrially satisfactory in view of optical purity of the resultant 3-aminopiperidine.

Patent Document 1: International Publication No. WO 2007/75630

### DISCLOSURE OF THE INVENTION

Under these circumstances, the present inventors have intensively studied a method of obtaining 3-aminopiperidine having higher optical purity and have found that an alkylpiperidin-3-yl carbamate and 3-aminopiperidine, each having high optical purity, or salts thereof can be obtained by optical resolution of an alkylpiperidin-3-yl carbamate having a structure in which an amino group at the 3-position of 3-aminopiperidine is protected with an alkoxycarbonyl group, using an optically active tartaric acid, thus leading to the present invention.

The present invention provides inventions described in the following [1] to [18]:
[1] A method for optical resolution of an alkylpiperidin-3-yl carbamate, the method including bringing an RS mixture of an alkylpiperidin-3-yl carbamate represented by the formula (1): 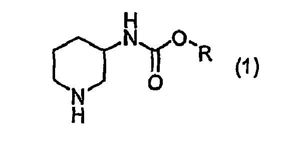 wherein R represents an alkyl group having 2 to 4 carbon atoms, into contact with an optically active tartaric acid in the presence of a solvent;
[2] The method for optical resolution according to [1], wherein the optically active tartaric acid is L-tartaric acid;
[3] A method for producing an optically active alkylpiperidin-3-yl carbamate represented by the formula (3): 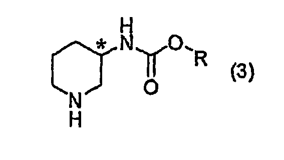 or a salt thereof,
   wherein R and * are as defined below, the method including bringing an RS mixture of an alkylpiperidin-3-yl carbamate
   represented by the formula (1): 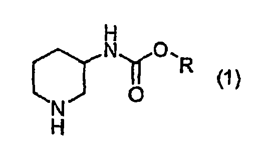 wherein R represents an alkyl group having 2 to 4 carbon atoms, into contact with an optically active tartaric acid in the presence of a solvent to crystallize a diastereomer salt of an optically active alkylpiperidin-3-yl carbamate represented by the formula (2): 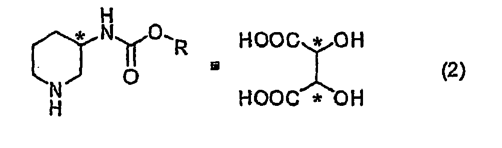 wherein R is as defined above and * represents that the carbon atom is an optically active center, and an optically active tartaric acid; and then reacting the diastereomer salt with an acid or a base at 0°C or higher and lower than 60°C;
[4] The production method according to [3], wherein the optically active tartaric acid is L-tartaric acid and the resultant optically active alkylpiperidin-3-yl carbamate represented by the formula (3) or a salt thereof is the R-form;
[5] A method for producing an optically active 3-aminopiperidine or a salt thereof, the method including' bringing an RS mixture of an alkylpiperidin-3-yl carbamate represented by the formula (1): 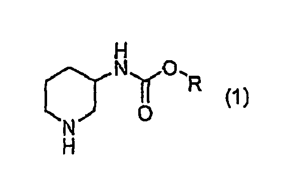 wherein R represents an alkyl group having 2 to 4 carbon atoms, into contact with an optically active tartaric acid in the presence of a solvent to crystallize a diastereomer salt of an optically active alkylpiperidin-3-yl carbamate represented by the formula (2): 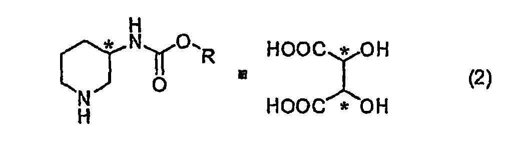 wherein R is as defined above and * represents that the carbon atom is an optically active center, and an optically active tartaric acid; and then reacting the diastereomer salt with an acid or a base at 60°C or higher and 150°C or lower;
[6] The production method according to [5], wherein the optically active tartaric acid is L-tartaric acid and the resultant optically active 3-aminopiperidine or a salt thereof is the R-form;
[7] The production method according to any one of [3] to [6], wherein R is an ethyl group, and the solvent is a mixed solvent of methanol and an alcohol having 2 to 4 carbon atoms;
[8] The production method according to any one of [3] to [6], wherein R is an ethyl group, and the solvent is a mixed solvent of methanol and 1-butanol;
[9] The production method according to any one of [3] to [6], wherein R is an alkyl group having 3 or 4 carbon atoms, and the solvent is at least one alcohol solvent selected from alcohols having 1 to 4 carbon atoms;
[10] The production method according to any one of [3] to [6], wherein R is an alkyl group having 3 or 4 carbon atoms, and the solvent is a mixed solvent of methanol and an alcohol having 2 to 4 carbon atoms, or an alcohol having 2 to 4 carbon atoms;
[11] The production method according to any one of [3] to [6], wherein R is a propyl group, and the solvent is ethanol;
[12] The production method according to any one of [3] to [6], wherein R is an isopropyl group, and the solvent is ethanol, 2-propanol, 1-butanol, or a mixed solvent of methanol and 1-butanol;
[13] The production method according to any one of [3] to [6], wherein R is an isobutyl group, and the solvent is a mixed solvent of methanol and 1-butanol, or ethanol;
[14] A diastereomer salt of an optically active alkylpiperidin-3-yl carbamate represented by the formula (2): 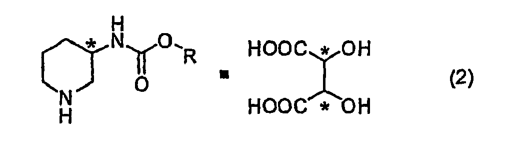 wherein R represents an alkyl group having 2 to 4 carbon atoms and * represents that the carbon atom is an optically active center, and an optically active tartaric acid;
[15] A diastereomer salt of an (R)-alkylpiperidin-3-yl carbamate represented by the formula (2a): 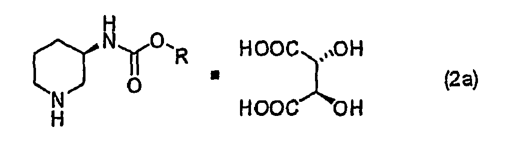 wherein R represents an alkyl group having 2 to 4 carbon atoms, and L-tartaric acid;
[16] An optically active alkylpiperidin-3-yl carbamate represented by the formula (3): 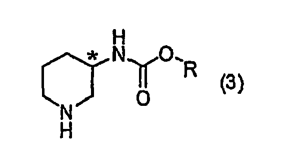 wherein R represents an alkyl group having 2 to 4 carbon atoms and * represents that the carbon atom is an optically active center;
[17] An (R)-alkylpiperidin-3-ylcarbamate represented by the formula (3a): 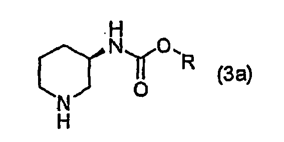 wherein R represents an alkyl group having 2 to 4 carbon atoms; and
[18] An alkylpiperidin-3-yl carbamate represented by the formula (1): 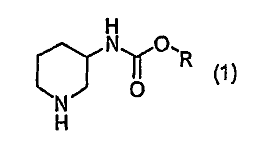 wherein R represents a propyl group or an isopropyl group.

### MODE FOR CARRYING OUT THE INVENTION

The present invention will be described in detail below.

In an alkylpiperidin-3-yl carbamate represented by the formula (1): 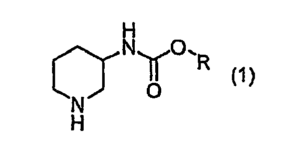 wherein R represents an alkyl group having 2 to 4 carbon atoms (hereinafter sometimes abbreviated as a carbamate compound (1)), examples of the alkyl group having 2 to 4 carbon atoms represented by R include an ethyl group, a propyl group, an isopropyl group, a butyl group, an isobutyl group, a s-butyl group and a t-butyl group. Examples of the carbamate compound (1) include ethyl piperidin-3-yl carbamate, propyl piperidin-3-yl carbamate, isopropyl piperidin-3-yl carbamate, butyl piperidin-3-yl carbamate, isobutyl piperidin-3-yl carbamate, s-butyl piperidin-3-yl carbamate and t-butyl piperidin-3-yl carbamate. Propyl piperidin-3-yl carbamate and isopropyl piperidin-3-yl carbamate are novel compounds.

An RS mixture of a carbamate compound (1) can be produced by any known method. For examples, the RS mixture may be produced by nuclear reduction of a carbamate compound (1) obtained by alkyl carbamation of an amino group at the 3-position of 3-aminopyridine (see U.S. Patent Application Publication No. 2005/0159423 Specification), or alkyl carbamation of an amino group at the 3-position of an RS mixture of 3-aminopiperidine.

The RS mixture of the carbamate compound (1) may be a mixture including both of R- and S-forms of an enantiomer thereof, but is usually a racemate. The RS mixture of the carbamate compound (1) may also be a salt with any acid other than tartaric acid. When the RS mixture of the carbamate compound (1) is used as a salt, the RS mixture of the carbamate compound (1) is preferably used after being freed from the RS mixture of the carbamate compound (1) by a treatment of bringing the salt into contact with the base.

A commercially available optically active tartaric acid can be usually used as the optically active tartaric acid. The optically active tartaric acid may also be a mixture thereof as well as D-tartaric acid and L-tartaric acid as long as any one of them is included in a significantly large amount. The optical purity is preferably 90% ee or more, more preferably 95% ee or more, still more preferably 98% ee or more, and most preferably 100% ee or more. When the R-form is desired as the resultant optically active alkylpiperidin-3-yl carbamate represented by the formula (3): 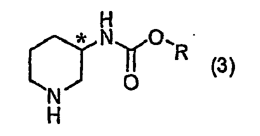 wherein R represents an alkyl group having 2 to 4 carbon atoms and * represents that the carbon atom is an optically active center (hereinafter sometimes abbreviated as an optically active carbamate compound (3)) or an optically active 3-aminopiperidine, L-tartaric acid is preferably used as the optically active tartaric acid. When the S-form is desired as the optically active carbamate compound (3) or optically active 3-aminopiperidine, D-tartaric acid is preferably used as the optically active tartaric acid.

The amount of the optically active tartaric acid used is not particularly limited as long as it is 1 mol or more based on 1 mol of the compound (the R-form when L-tartaric acid is used, or the S-form when D-tartaric acid is used) in which formation of a salt with the optically active tartaric acid is desired, among the RS mixture of the carbamate compound (1). When a racemate is used as the RS mixture of the carbamate compound (1), the amount of the optically active tartaric acid used may be usually from 0.5 mol or more based on 1 mol of the racemate. From the viewpoints of yield and economical efficiency, the amount of the optically active tartaric acid used is preferably from 0.9 to 2 mol, and more preferably from 1.0 to 1.5 mol.

Contact between the RS mixture of the carbamate compound (1) with the optically active tartaric acid is usually conducted in the presence of a solvent. Examples of the solvent include aliphatic hydrocarbon solvents such as pentane, hexane, isohexane, heptane, isoheptane, octane, isooctane, nonane, isononane, decane, isodecane, undecane, dodecane, cyclopentane, cyclohexane, methylcyclohexane, t-butylcyclohexane and petroleum ether; aromatic solvents such as benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene and 1,2,4-trichlorobenzene; ether solvents such as tetrahydrofuran, methyltetrahydrofuran, diethylether, dipropyl ether, diisopropyl ether, dibutyl ether, dipentyl ether, dihexyl ether, diheptyl ether, dioctyl ether, t-butyl methyl ether, cyclopentyl methyl ether, 1,2-dimethoxyethane, diethylene glycol dimethyl ether, anisole and diphenyl ether; alcohol solvents such as methanol, ethanol, 1-propanol, 2-propanol, 1-butanol, isobutyl alcohol, t-butyl alcohol, 1-pentanol, 2-pentanol, isopentyl alcohol, 1-hexanol, 2-hexanol, isohexyl alcohol, 1-heptanol, 2-heptanol, 3-heptanol, isoheptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether and diethylene glycol mono-t-butyl ether; nitrile solvents such as acetonitrile, propionitrile and benzonitrile; chlorinated aliphatic hydrocarbon solvents such as dichloromethane, chloroform and 1,2-dichloroethane; aprotic polar solvents such as dimethyl sulfoxide, sulfolane, N,N-dimethylformamide, N,N-dimethylacetamide, N,N-dimethylpropionamide, N-methyl pyrrolidone, γ-butyrolactone, methyl carbonate, ethyl carbonate, ethylene carbonate, propylene carbonate, 1,3-dimethyl-2-imidazolidinone, 1,3-dimethyl-3,4,5,6-tetrahydro-2(1H)-pyridinone and acetone; carboxylic acid solvents such as formic acid, acetic acid and propionic acid; and water. These solvents may be used alone, or two or more kinds of them may be simultaneously used.

Among these solvents, alcohol solvents are preferable, and an alcohol having 1 to 4 carbon atoms alone or a mixed solvent is more preferable from the viewpoints of optical purity and yield. When R in the above formula (1) is an ethyl group, a mixed solvent of methanol and an alcohol having 2 to 4 carbon atoms, or methanol is preferable, a mixed solvent of methanol and 1-butanol, or methanol is more preferable, and methanol is still more preferable. In this case, the content of methanol in the solvent is usually from 1 to 100% (volume/volume), preferably from 10 to 100% (volume/volume), and more preferably from 40 to 100% (volume/volume). When R in the above formula (1) is an alkyl group having 3 or 4 carbon atoms, at least one alcohol solvent selected from an alcohol having 1 to 4 carbon atoms is preferable, and a mixed solvent of methanol and an alcohol having 2 to 4 carbon atoms, or an alcohol having 2 to 4 carbon atoms is more preferable. When R in the above the formula (1) is a propyl group, ethanol is preferable. When R in the above the formula (1) is an isopropyl group, a mixed solvent of methanol and 1-butanol, ethanol, 2-propanol or 1-butanol is preferable. When R in the above the formula (1) is an isobutyl group, a mixed solvent of methanol and 1-butanol, or ethanol is preferable. When R in the above the formula (1) is an alkyl group having 3 or 4 carbon atoms, the content of methanol in a mixed solvent of methanol and an alcohol having 2 to 4 carbon atoms is usually from 1 to 99% (volume/volume), preferably from 10 to 90% (volume/volume), and more preferably from 30 to 70% (volume/volume).

The amount of the solvent used may be appropriately selected according to solubility of the resultant diastereomer salt (hereinafter sometimes abbreviated as a diastereomer salt (2)) of an optically active alkylpiperidin-3-yl carbamate represented by the formula (2): 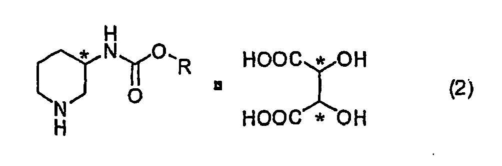 wherein R represents an alkyl group having 2 to 4 carbon atoms and * represents that the carbon atom is an optically active center, and an optically active tartaric acid. The amount of the solvent used is usually from 1 to 50 L, and preferably from 3 to 30 L, based on 1 kg of the RS mixture of the carbamate compound (1).

Contact of the RS mixture of the carbamate compound (1) with the optically active tartaric acid is conducted by mixing them in the presence of a solvent, and the order of mixing is not particularly limited. When a crystal of the diastereomer salt (2) does not exist in the resultant mixture, the diastereomer salt (2) may be crystallized by subjecting the mixture to a cooling treatment with or without adding the crystal of the diastereomer salt as a seed crystal. When the crystal of the diastereomer salt (2) exists in the mixture obtained by the contact, the mixture may be directly subjected to a cooling treatment. However, from the viewpoints of chemical purity and optical purity of the finally obtained optically active carbamate compound (3) or optically active 3-aminopiperidine or salts thereof, it is preferred that the diastereomer salt (2) be crystallized by being subjected to a cooling treatment after dissolving the crystal of the diastereomer salt (2) by heating the mixture. In the crystallization of the diastereomer salt (2), a seed crystal of the diastereomer salt (2) may also be used.

The temperature at which the RS mixture of the carbamate compound (1) and the optically active tartaric acid are mixed is not particularly limited, and is usually within a range of 0°C or higher and a boiling point of the solvent or lower. In the case of heating after mixing them, the mixture is usually heated within a range of 30°C or higher and a boiling point of the solvent or lower. The cooling temperature is usually within a range from 0 to 25°C, and it is preferred to gradually cool from the viewpoints of chemical purity and optical purity of the resultant diastereomer salt (2).

It is possible to take out the diastereomer salt (2) as a solid by subjecting the mixture thus obtained to a solid-liquid separation treatment such as filtration or decantation. Such a diastereomer salt (2) is a novel compound. The liquid obtained by the above solid-liquid separation treatment usually contains a carbamate compound (1) rich in an enantiomer reverse to that constituting the diastereomer salt (2), and it is also possible to take out the carbamate compound (1) rich in an enantiomer reverse to that constituting the diastereomer salt (2), as an optically active carbamate compound (3) or a salt thereof, from the liquid by a conventional method.

The diastereomer salt (2) thus taken out may be directly reacted with an acid or a base. However, from the viewpoints of chemical purity and optical purity of the finally obtained optically active carbamate compound (3) or optically active 3-aminopiperidine, it is preferred that the diastereomer salt be treated with an acid or a base after being subjected to a washing treatment. In the washing treatment, the same solvents as those described above can be usually used. It is preferred that a drying treatment be conducted after washing. The drying treatment is usually conducted at a temperature within a range from 20 to 80°C under the conditions of a normal pressure or reduced pressure.

The diastereomer salts (2) thus obtained vary depending on steric configuration of the optically active tartaric acid used, and are usually a diastereomer salt (hereinafter sometimes abbreviated as a diastereomer salt (2a)) of an (R)-alkylpiperidin-3-yl carbamate represented by the formula (2a): 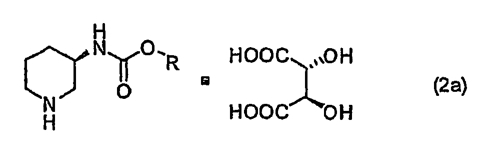 wherein R represents an alkyl group having 2 to 4 carbon atoms, and L-tartaric acid, and a diastereomer salt of an (S)-alkylpiperidin-3-yl carbamate represented by the formula (2b) : 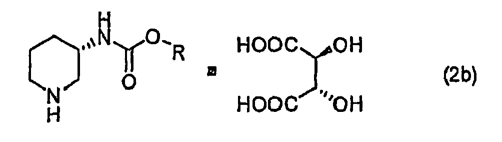 wherein R represents an alkyl group having 2 to 4 carbon atoms, and D-tartaric acid.

An optically active carbamate compound (3) or a salt thereof is preferentially obtained by reacting the diastereomer salt (2) with an acid or a base at a temperature lower than 60°C usually, while an optically active 3-aminopiperidine or a salt thereof is preferentially obtained by reacting with an acid or a base at 60°C or higher.

Here, a method of producing an optically active carbamate compound (3) or a salt thereof by reacting the diastereomer salt (2) with an acid or a base at a temperature lower than 60°C will be described below.

The acid to be reacted with the diastereomer salt (2) may be an acid having stronger acidity than that of tartaric acid, and examples thereof include mineral acids such as hydrochloric acid, phosphoric acid and sulfuric acid; and organic acids such as paratoluenesulfonic acid, benzenesulfonic acid and camphorsulfonic acid. Hydrochloric acid is preferable. A commercially available acid can be used for the acid as it is, and can also be used as a solution of a solvent described hereinafter.

The amount of the acid used is not particularly limited as long as it is 1 mol or more based on 1 mol of the optically active carbamate compound (3) constituting the diastereomer salt (2).

The diastereomer salt (2) is usually reacted with the acid in the presence of a solvent. Examples of the solvent include aliphatic hydrocarbon solvents such as pentane, hexane, isohexane, heptane, isoheptane, octane, isooctane, nonane, isononane, decane, isodecane, undecane, dodecane, cyclopentane, cyclohexane, methylcyclohexane, t-butylcyclohexane and petroleum ether; aromatic solvents such as benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene and 1,2,4-trichlorobenzene; ether solvents such as tetrahydrofuran, methyltetrahydrofuran, diethyl ether, dipropyl ether, diisopropyl ether, dibutyl ether, dipentyl ether, dihexyl ether, diheptyl ether, dioctyl ether, t-butyl methyl ether, cyclopentyl methyl ether, 1,2-dimethoxyethane, diethylene glycol dimethyl ether, anisole and diphenyl ether; alcohol solvents such as methanol, ethanol, 1-propanol, 2-propanol, 1-butanol, isobutyl alcohol, t-butyl alcohol, 1-pentanol, 2-pentanol, isopentyl alcohol, 1-hexanol, 2-hexanol, isohexyl alcohol, 1-heptanol, 2-heptanol, 3-heptanol, isoheptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether and diethylene glycol mono-t-butyl ether; nitrile solvents such as acetonitrile, propionitrile and benzonitrile; ester solvents such as ethyl acetate, propyl acetate, isopropyl acetate, butyl acetate, isobutyl acetate, t-butyl acetate, amyl acetate and isoamyl acetate; ketone solvents such as acetone, methyl ethyl ketone, methyl isopropyl ketone, methyl isobutyl ketone, cyclopentanone and cyclohexanone; chlorinated aliphatic hydrocarbon solvents such as dichloromethane, chloroform and 1,2-dichloroethane; carboxylic acid solvents such as formic acid, acetic acid and propionic acid; and water. These solvents may be used alone, or two or more kinds of them may be simultaneously used. Among these solvents, aromatic solvents, alcohol solvents or water are preferable, toluene, xylene, methanol, ethanol, 2-propanol, 1-propanol, 1-butanol or water is more preferable, and 1-butanol or water is still more preferable.

The amount of the solvent used is usually from 1 to 50 L, and preferably from 3 to 30 L, based on 1 kg of the diastereomer salt (2).

The diastereomer salt (2) and the acid may be usually mixed at 0 to 60°C, and preferably 10 to 30°C, and the order of mixing them is not particularly limited.

The reaction time is not particularly limited, and is usually within a range from 1 minute to 24 hours.

When a salt of the optically active carbamate compound (3) and the acid used is precipitated in the resultant mixture, the salt can be taken out by directly subjecting the mixture to a solid-liquid separation treatment such as filtration or decantation. When the salt is not sufficiently precipitated or the salt is not precipitated, the salt may be taken out by concentrating the mixture, mixing the mixture with a solvent, which does not easily dissolve the salt, or heating or cooling the mixture thereby to crystallize the salt, and subjecting the resultant mixture to a solid-liquid separation treatment such as filtration or decantation. The resultant salt may be further purified by conventional means such as recrystallization, or may be freed in the same manner as in the case of reacting a base described hereinafter. The filtrate obtained by the above solid-liquid separation treatment usually contains an optically active tartaric acid, and it is possible to recover an optically active tartaric acid from the filtrate by a conventional method and recycle it in an optical resolution method or a production method of the present invention.

Examples of the base to be reacted with the diastereomer salt (2) include alkali metal hydroxides such as potassium hydroxide and sodium hydroxide; alkali metal carbonates such as sodium carbonate and potassium carbonate; and alkali metal alcoholates such as sodium methylate, sodium ethylate, potassium methylate and potassium ethylate. Alkali metal carbonates are preferable, and sodium carbonate is more preferable. A commercially available base can be directly used as the base, and also can be used as a solution of a solvent described hereinafter.

The amount of the base used is not particularly limited as long as it is 1 mol or more based on 1 mol of the optically active tartaric acid constituting the diastereomer salt (2).

The diastereomer salt (2) is usually reacted with a base in the presence of a solvent. Examples of the solvent include alcohol solvents such as methanol, ethanol, 2-propanol, 1-propanol and n-butyl alcohol; ether solvents such as diethyl ether, t-butyl methyl ether, methyl isobutyl ether, diisopropyl ether, methyl cyclopentyl ether and 1,2-dimethoxymethane; aromatic solvents such as toluene, xylene and chlorobenzene; aliphatic hydrocarbon solvents such as hexane and cyclohexane; ketone solvents such as methyl ethyl ketone and methyl isobutyl ketone; ester solvents such as ethyl acetate and t-butyl acetate; halogenated aliphatic hydrocarbon solvents such as dichloromethane; and water. These solvents may be used alone, or two or more kinds of them may be simultaneously used. When inorganic bases such as alkali metal hydroxides and alkali metal carbonates are used as the base, it is preferred to use water alone or to simulatneously use organic solvents having low compatibility with water (ether solvents, aromatic solvent, aliphatic hydrocarbon solvents, ketone solvents, ester solvents and halogenated hydrocarbon solvents described above) and water. When alkali metal alcoholates are used as the base, alcohol solvents are preferable.

The amount of the solvent used is usually from 1 to 50 L, and preferably from 3 to 30 L, based on 1 kg of the diastereomer salt (2).

The diastereomer salt (2) and the base may be usually mixed at 0 to 60°C, and preferably 10 to 30°C, and the order of mixing them is not particularly limited.

The reaction time is not particularly limited, and is usually within a range from 1 minute to 24 hours.

For example, an organic layer containing an optically active carbamate compound (3) can be obtained by adding a base to a mixture of water and a diastereomer salt (2) thereby to make an aqueous layer of the mixture alkaline (usually pH 8.5 or more), reacting them at a predetermined temperature, and adding an organic solvent having low compatibility with water to the resultant mixture, followed by a liquid separation treatment, and then an optically active carbamate compound (3) can be isolated by optionally washing the organic layer with water and subjecting it to a concentration treatment. An alkali metal salt of an optically active tartaric acid is usually precipitated when an alkali metal alcoholate is used as the base and alcohol solvents are used as the solvent, and then an optically active carbamate compound (3) can be isolated by removing the alkali metal salt through filtration and subjecting the resultant solution to a concentration treatment. The resultant optically active carbamate compound (3) may be further purified, for example, by conventional means such as rectification, recrystallization or column chromatography. The optically active carbamate compound (3) can also be taken out as an acid addition salt. The aqueous layer obtained by the above liquid separation treatment contains an optically active tartaric acid, and it is possible to recover an optically active tartaric acid from the aqueous layer and recycle it in the present invention. It is also possible to recover an optically active tartaric acid by a conventional method from the alkali metal salt of the optically active tartaric acid removed by filtration and recycle it in an optical resolution method or a production method of the present invention.

The optically active carbamate compound (3) thus obtained is an optically active carbamate compound (3) constituting the diastereomer salt (2) subjected to the treatment. That is, when the diastereomer salt (2a) is subjected to the treatment, it is possible to obtain an (R)-alkylpiperidin-3-yl carbamate represented by the formula (3a): 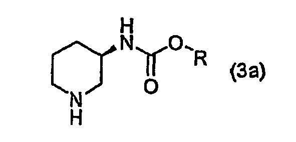 wherein R represents an alkyl group having 2 to 4 carbon atoms. The optical purity of the optically active carbamate compound (3) is usually from 90% ee or more, although it varies depending on the optical purity of the optically active tartaric acid used.

It is also possible to obtain an optically active 3-aminopiperidine or a salt thereof by reacting the resultant optically active carbamate compound (3) or a salt thereof with an acid or a base at 60°C or higher in accordance with a method which will be described below.

A method of producing an optically active 3-aminopiperidine or a salt thereof by reacting a diastereomer salt with an acid or a base at 60°C or higher will be described below.

The acid to be reacted with the diastereomer salt (2) may be a strong acid with pH 1 or less, and examples thereof include mineral acids such as hydrochloric acid, phosphoric acid and sulfuric acid; and organic acids such as paratoluenesulfonic acid, benzenesulfonic acid and camphorsulfonic acid. Hydrochloric acid is preferable. A commercially available acid can be used for the acid as it is, and can also be used as a solution of a solvent described hereinafter.

The amount of the acid used is not particularly limited as long as it is 1.1 mol or more based on 1 mol of the optically active carbamate compound (3) constituting the diastereomer salt (2).

The diastereomer salt (2) is usually reacted with the acid in the presence of a solvent. Examples of the solvent include aliphatic hydrocarbon solvents such as pentane, hexane, isohexane, heptane, isoheptane, octane, isooctane, nonane, isononane, decane, isodecane, undecane, dodecane, cyclopentane, cyclohexane, methylcyclohexane, t-butylcyclohexane and petroleum ether; aromatic solvents such as benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene and 1,2,4-trichlorobenzene; ether solvents such as tetrahydrofuran, methyltetrahydrofuran, diethyl ether, dipropyl ether, diisopropyl ether, dibutyl ether, dipentyl ether, dihexyl ether, diheptyl ether, dioctyl ether, t-butyl methyl ether, cyclopentyl methyl ether, 1,2-dimethoxyethane, diethylene glycol dimethyl ether, anisole and diphenyl ether; alcohol solvents such as methanol, ethanol, 1-propanol, 2-propanol, 1-butanol, isobutyl alcohol, t-butyl alcohol, 1-pentanol, 2-pentanol, isopentyl alcohol, 1-hexanol, 2-hexanol, isohexyl alcohol, 1-heptanol, 2-heptanol, 3-heptanol, isoheptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether and diethylene glycol mono-t-butyl ether; ketone solvents such as acetone, methyl ethyl ketone, methyl isopropyl ketone, methyl isobutyl ketone, cyclopentanone and cyclohexanone; chlorinated aliphatic hydrocarbon solvents such as dichloromethane, chloroform and 1,2-dichloroethane; carboxylic acid solvents such as formic acid, acetic acid and propionic acid; and water. These solvents may be used alone, or two or more kinds of them may be simultaneously used. Of these solvents, aromatic solvents, alcohol solvents or water are preferable, toluene, xylene, methanol, ethanol, 2-propanol, 1-propanol, 1-butanol or water is more preferable, and water or 1-butanol is still more preferable.

The amount of the solvent used is usually from 1 to 50 L, and preferably from 3 to 30 L, based on 1 kg of the diastereomer salt (2).

The diastereomer salt (2) and the acid may be usually mixed at 60 to 150°C, and preferably 80 to 120°C, and the order of mixing them is not particularly limited.

The reaction time is not particularly limited, and is usually within a range from 1 to 24 hours.

When a salt of the optically active 3-aminopiperidine and the acid used is precipitated in the resultant mixture, the salt can be taken out by directly subjecting the mixture to a solid-liquid separation treatment such as filtration or decantation. When the salt is not sufficiently precipitated or the salt is not precipitated, the salt may be taken out by concentrating the mixture, mixing the mixture with a solvent, which does not easily dissolve the salt, or heating or cooling the mixture thereby to crystallize the salt, and subjecting the resultant mixture to a solid-liquid separation treatment such as filtration or decantation. The resultant salt may be further purified by conventional means such as recrystallization, or may be freed in the same manner as in the case of a base treatment described hereinafter. The filtrate obtained by the above solid-liquid separation treatment usually contains an optically active tartaric acid, and it is possible to recover an optically active tartaric acid from the filtrate by a conventional method and recycle it in the present invention.

It is possible to show, as a preferred embodiment so as to obtain an optically active 3-aminopiperidine or a salt thereof by reacting the diastereomer salt (2) with the acid, a method in which the diastereomer salt (2) is reacted with hydrochloric acid at 80 to 120°C in the presence of water and, after distilling off water, a crystal is precipitated by mixing the resultant mixture with an organic solvent such as 1-butanol, and then an optically active 3-aminopiperidine is taken as a dihydrochloride by collecting the crystal through filtration and drying the crystal. Here, a solvent such as 1-butanol, which undergoes azeotropic distillation with water, may be used so as to efficiently distill off water after reacting the diastereomer salt (2) with hydrochloric acid. The solvent, which undergoes azeotropic distillation with water, may be used in advance when the diastereomer salt (2) is reacted with water. When the solvent remains in the mixture after distilling off water, an operation of mixing the mixture with an organic solvent, which is conducted to precipitate a crystal, may be omitted.

Examples of the base to be reacted with the diastereomer salt (2) include alkali metal hydroxides such as potassium hydroxide and sodium hydroxide; alkali metal carbonates such as sodium carbonate and potassium carbonate; and alkali metal alcoholates such as sodium methylate, sodium ethylate, potassium methylate and potassium ethylate. Alkali metal hydroxides are preferable, and sodium hydroxide is more preferable.

The diastereomer salt (2) is usually reacted with a base in the presence of a solvent. Examples of the solvent include alcohol solvents such as methanol, ethanol, 2-propanol, 1-propanol and 1-butanol; ether solvents such as diethyl ether, t-butyl methyl ether, methyl isobutyl ether, diisopropyl ether, methyl cyclopentyl ether and 1,2-dimethoxymethane; aromatic solvents such as toluene, xylene and chlorobenzene; aliphatic hydrocarbon solvents such as hexane and cyclohexane; ketone solvents such as methyl ethyl ketone and methyl isobutyl ketone; ester solvents such as ethyl acetate and t-butyl acetate; halogenated aliphatic hydrocarbon solvents such as dichloromethane; and water. These solvents may be used alone, or two or more kinds of them may be simultaneously used. When inorganic bases such as alkali metal hydroxides and alkali metal carbonates are used as the base, it is preferred to use water alone or to simultaneously use organic solvents having low compatibility with water (ether solvents, aromatic solvent, aliphatic hydrocarbon solvents, ketone solvents, ester solvents and halogenated hydrocarbon solvents described above) and water. When alkali metal alcoholates are used as the base, alcohol solvents are preferable.

The amount of the solvent used is usually from 1 to 50 L, and preferably from 3 to 30 L, based on 1 kg of the diastereomer salt (2).

The diastereomer salt (2) and a base may be usually mixed at 60 to 150°C, and preferably 80 to 120°C, and the order of mixing them is not particularly limited.

The reaction time is not particularly limited, and is usually within a range from 1 to 24 hours.

For example, an organic layer containing an optically active 3-aminopiperidine can be obtained by adding a base to a mixture of water and a diastereomer salt (2) thereby to make an aqueous layer of the mixture alkaline (usually pH 13 or more), reacting at a predetermined temperature, and adding an organic solvent having low compatibility with water to the resultant mixture, followed by a liquid separation treatment, and then an optically active 3-aminopiperidine can be isolated by optionally washing the organic layer with water and subjecting it to a concentration treatment. An alkali metal salt of an optically active tartaric acid is usually precipitated when an alkali metal alcoholate is used as the base and alcohol solvents are used as the solvent, and then an optically active 3-aminopiperidine can be isolated by removing the alkali metal salt through filtration and subjecting the resultant solution to a concentration treatment. The resultant optically active 3-aminopiperidine may be further purified, for example, by conventional means such as rectification, recrystallization or column chromatography. The optically active 3-aminopiperidine can also be taken out as an acid addition salt. The aqueous layer obtained by the above liquid separation treatment contains an optically active tartaric acid, and it is possible to recover an optically active tartaric acid from the aqueous layer by a conventional method and recycle it in the present invention. It is also possible to recover an optically active tartaric acid by a conventional method from the alkali salt of the optically active tartaric acid removed by filtration and recycle it in an optical resolution method or a production method of the present invention.

Steric configuration of the optically active 3-aminopiperidine thus obtained corresponds to an optically active carbamate compound (3) constituting the diastereomer salt (2) subjected to a treatment. That is, when the diastereomer salt (2a) is subjected to the treatment, (R)-3-aminopiperidine is obtained. The optical purity of the optically active 3-aminopiperidine is usually 90% ee or more, although it varies depending on the optical purity of the optically active tartaric acid used.

The present invention can provide an alkylpiperidin-3-yl carbamate and 3-aminopiperidine, each having high optical purity, or salts thereof, and is therefore industrially advantageous.

### Examples

The present invention will be described in more detail below by way of Examples, but the present invention is not limited to these Examples.

### Production Example 1: Production of isopropylpiperidin-3-yl carbamate (racemate)

To a solution prepared by dissolving 50.0 g (0.53 mol) of 3-aminopyridine and 8.94 g (0.11 mol) of sodium hydrogen carbonate in 150 ml of water, 75.7 g (0.61 mol) of isopropyl chlorocarbonate and 230 ml of an aqueous 15 wt% potassium hydroxide solution were simultaneously added dropwise over 2 hours. During the dropwise addition, the mixture was maintained at the inner temperature of 0 to 10°C and pH of 7 to 8. After completion of the dropwise addition, the resultant mixture was stirred at room temperature for 1 hour, and the precipitated crystal was filtered and then washed with 200 ml of water. The resultant crystal was dried to obtain 89.3 g of isopropylpyridin-3-yl carbamate. Yield: 93.3%.

In a solution prepared by dissolving 89.3 g of the resultant isopropylpyridin-3-yl carbamate in 178.6g (2.97 mol) of acetic acid, 17.9 g of palladium carbon (5%) was charged, followed by stirring under a hydrogen pressure of 0.5 Mpa at 70°C for 14 hours. After the completion of the reaction, palladium carbon was removed by filtration to obtain the reaction solution and palladium carbon was washed with 225 ml of water to obtain a wash, and then the reaction solution was mixed with the wash. The resultant solution was added dropwise in a solution prepared by dissolving 119 g (2.98 mol) of sodium hydroxide in 129 ml of water in advance. During the dropwise addition, the mixture was maintained at the inner temperature of 0 to 10°C. The resultant mixture was extracted with 180 ml of t-butyl methyl ether and the resultant organic layer was dried over magnesium sulfate. Magnesium sulfate was removed by filtration and the resultant solution was concentrated to obtain 85.0 g of isopropylpiperidin-3-yl carbamate as a yellowish white crystal. Yield: 92.1% (based on isopropylpyridin-3-yl carbamate). ¹H-NMR (DMSO-d₆, 400MHz) δ ppm 6.85 (1H, d, J=7.8Hz), 4.74-4.68 (1H, m), 3.30-3.15 (1H, m), 2.85 (1H, d-like, J=11.7Hz), 2.69 (1H, d-like, J=12.2Hz), 2.30 (1H, t-like, J=10.2Hz), 2.19 (1H, t-like, J=11.2Hz), 2.10-1.95 (1H, m), 1.80-1.68 (1H, m), 1.58-1.49 (1H, m), 1.35-1.18 (2H, m), 1.14 (6H, d, J=6.3 Hz) ¹³C-NMR (DMSO-d₆, 400MHz) δ ppm: 155.0, 66.2, 51.6, 47.8, 45.6, 31.0, 25.2, 22.1

### Production Example 2: Production of ethylpiperidin-3-yl carbamate (racemate)

In a solution prepared by dissolving 100 g (1.06 mol) of 3-aminopyridine in 650 ml of water, 121.1 g (1.12 mol) of ethyl chlorocarbonate and 240 g of an aqueous 20 wt% sodium hydroxide solution were simultaneously added dropwise over 2 hours.

During the dropwise addition, the mixture was maintained at the inner temperature of 0 to 15°C and pH of 7 to 8.5. After completion of the dropwise addition, the resultant mixture was stirred at 10°C for 2 hours, and the precipitated crystal was filtered and then washed with 500 ml of water. The resultant crystal was dried to obtain 148.5 g of ethylpyridin-3-yl carbamate. Yield: 84.1%.

In a solution prepared by dissolving 145 g of the resultant ethylpyridin-3-yl carbamate in 157 g (2.61 mol) of acetic acid and 145 ml of water, 14.5 g of palladium carbon (10%) was charged, followed by stirring under a hydrogen pressure of 0.5 Mpa at 70 to 95°C for 6 hours. After the completion of the reaction, palladium carbon was removed by filtration to obtain the reaction solution and palladium carbon was washed with 300 ml of 1-butanol to obtain a wash, and then the reaction solution was mixed with the wash. To the resultant solution, a solution prepared by dissolving 105 g (2.63 mol) of sodium hydroxide in 244 ml of water in advance was added dropwise. During the dropwise addition, the mixture was maintained at the inner temperature of 0 to 30°C. The resultant mixture was subjected to a liquid separation treatment and the organic layer was 100 g of an aqueous 20 wt% sodium chloride solution. Then, the organic layer was concentrated and dissolved in 500 ml of 2-propanol, and a solution obtained by removing insolubles through filtration was concentrated to obtain 141.9 g of ethylpiperidin-3-yl carbamate as a pale yellowish white crystal. Yield: 94.4% (based on ethylpyridin-3-yl carbamate).

### Production Example 3: Production of propylpiperidin-3-yl carbamate (racemate)

In a solution prepared by dissolving 42.2 g (0.45 mol) of 3-aminopyridine in 148 ml of water, 54.9g (0.45mol) of propyl chlorocarbonate and 100 g of an aqueous 20 wt% sodium hydroxide solution were simultaneously added dropwise over 2 hours. During the dropwise addition, the mixture was maintained at the inner temperature of 0 to 15°C and pH of 7 to 8.5. After completion of the dropwise addition, the resultant mixture was stirred at room temperature for 1 hour, and the precipitated crystal was filtered and then washed with 300 ml of water. The resultant crystal was dried to obtain 64.8 g of propylpyridin-3-yl carbamate. Yield: 80.3%.

In a solution prepared by dissolving 60.0 g of the resultant propylpyridin-3-yl carbamate in 100 g (1.67 mol) of acetic acid and 100 ml of water, 6.0 g of palladium carbon (5%) was charged, followed by stirring under a hydrogen pressure of 0.5 Mpa at 70 to 85°C for 8 hours. After the completion of the reaction, palladium carbon was removed by filtration to obtain the reaction solution, and palladium carbon was washed with 180 ml of 1-butanol to obtain a wash, and then the reaction solution was mixed with the wash. To the resultant solution, a solution prepared by dissolving 66.6 g (1.67 mol) of sodium hydroxide in 200 ml of water in advance was added dropwise. During the dropwise addition, the mixture was maintained at the inner temperature of 0 to 30°C. The resultant mixture was subjected to a liquid separation treatment and the organic layer was washed with 100 g of an aqueous 20 wt% sodium chloride solution. Then, the organic layer was concentrated and dissolved in 300 ml of 2-propanol, and a solution obtained by removing insolubles through filtration was concentrated to obtain 59.1 g of propylpiperidin-3-yl carbamate as a pale yellow oily product. Yield: 95.3% (based on propylpyridin-3-yl carbamate). ¹H-NMR (DMSO-d₆, 400MHz) δ ppm: 6.95 (1H, d, J=8Hz), 3.87 (2H, t, J=7Hz), 3.28-3.26 (1H, m), 2.88 (1H, d-like), 2.72 (1H, d-like), 2.33 (1H, t-like), 2.23 (1H, t-like), 1.76-1.74 (1H, m), 1.59-1.50 (3H, m), 1.34-1.24 (2H, m), 0.88 (3H, t, J=7Hz) ¹³C-NMR (DMSO-D₆, 400MHz) δ ppm: 155.5, 65.0, 51.5, 47.8, 45.6, 31.0, 25.2, 22.1, 10.3

### Production Example 4: Production of isobutylpiperidin-3-yl carbamate (racemate)

In a solution prepared by dissolving 16.4 g (0.17 mol) of 3-aminopyridine in 100 ml of water, 25.0 g (0.18 mol) of isobutyl chlorocarbonate and 100 g of an aqueous 15 wt% sodium hydroxide solution were simultaneously added dropwise over 2 hours. During the dropwise addition, the mixture was maintained at the inner temperature of 0 to 17°C and pH of 7 to 8.5. After completion of the dropwise addition, the resultant mixture was stirred overnight at room temperature, and the precipitated crystal was filtered and then washed with 300 ml of water. The resultant crystal was dried to obtain 31.2 g of isobutylpyridin-3-yl carbamate. Yield: 92.2%.

In a solution prepared by dissolving 30.4 g of the resultant isobutylpyridin-3-yl carbamate in 18.9 g (0.31 mol) of acetic acid, 45 ml of water and 90 ml of 1-butanol, 1.5 g of palladium carbon (5%) was charged, followed by stirring under a hydrogen pressure of 0.5 Mpa at 70 to 85°C for 5 hours. After the completion of the reaction, palladium carbon was removed by filtration to obtain the reaction solution and palladium carbon was washed with 30 ml of 1-butanol to obtain a wash, and then the reaction solution was mixed with the wash. To the resultant solution, a solution prepared by dissolving 12.6 g (0.31 mol) of sodium hydroxide in 29 ml of water was added dropwise. During the dropwise addition, the mixture was maintained at the inner temperature of 0 to 5°C. The resultant mixture was subjected to a liquid separation treatment and the organic layer was washed four times with 50 g of water. Then, the organic layer was concentrated to obtain 30.2 g of isobutylpiperidin-3-yl carbamate as a yellowish white crystal. Yield: 95.9% (based on isobutylpyridin-3-yl carbamate).

### Example 1: Optical resolution of isopropylpiperidin-3-yl carbamate

To a solution prepared by dissolving 10 g (53.7 mmol) of isopropylpiperidin-3-yl carbamate in 50 ml of ethanol in the same manner as in Production Example 1, 8.46 g (56.4 mmol) of L-tartaric acid was added and the resultant solution was stirred at 40°C, and then a seed crystal of a diastereomer salt of (R)-isopropylpiperidin-3-yl carbamate and L-tartaric acid was added to precipitate a crystal. The resultant mixture was heated to 65°C and stirred at the same temperature for 1 hour. As a result, most of the precipitated crystal was dissolved and a portion thereof remained without being dissolved. The resultant mixture was air-cooled to room temperature while stirring, cooled to 0 to 5°C and then stirred at the same temperature for 5 hours. A crystal was collected from the resultant mixture through filtration and the crystal was washed with 20 ml of cold ethanol. The resultant crystal was dried to obtain 6.95 g of a diastereomer salt of (R)-isopropylpiperidin-3-yl carbamate and L-tartaric acid as a white crystal. Yield: 38.5%.
¹H-NMR (DMSO-d₆, 400MHz) δ ppm: 7.24 (1H, d, J=8Hz), 4.80-4.72 (1H, m), 3.91 (2H, s), 3.60 (1H, br), 3.18 (1H, dd, J=4.12Hz), 3.09 (1H, d, J=13Hz), 2.72 (1H, t, J=11Hz), 2.59 (1H, t, J=12Hz), 1.82-1.77 (2H, m), 1.63-1.54 (1H, m), 1.43-1.31 (1H, m), 1.17 (6H, d, J=6Hz)
¹³C-NMR (DMSO-d6, 400MHz) δ ppm: 174.5, 155.0, 71.8, 66.9, 46.6, 44.6, 42.7, 28.4, 22.0, 20.7

Using triethylamine, isopropylpiperidin-3-yl carbamate was taken out from the diastereomer salt, derivatized by 3,5-dinitrobenzoyl chloride, and then analyzed by high-performance liquid chromatography. As a result, the optical purity of isopropylpiperidin-3-yl carbamate in the diastereomer salt was 93.5% ee (R-form).

### <Conditions for analysis of optical purity>

Column: CHIRALCEL AS-RH (4.6*150 mm, 5 µm)

Mobile phase: A = water, B = acetonitrile, A/B = 70/30 Flow rate: 1.0 ml/min

Detector: UV 254 nm

Retention time: S-form = 19.1 minutes, R-form = 31.5 minutes

### Example 2: Optical resolution of isopropylpiperidin-3-yl carbamate

To a solution prepared by dissolving 3.0 g (16.1 mmol) of isopropylpiperidin-3-yl carbamate in 15 ml of a mixed solvent of methanol and 1-butanol (1/1 (volume/volume)) in the same manner as in Production Example 1, 2.78 g (18.5 mmol) of L-tartaric acid was added and the resultant solution was stirred at 40°C, and then a seed crystal of a diastereomer salt of (R)-isopropylpiperidin-3-yl carbamate and L-tartaric acid was added to precipitate a crystal. The resultant mixture was stirred at the same temperature for 1 hour, air-cooled to room temperature while stirring, and then stirred at the same temperature for 3 hours. The mixture was cooled to 10°C and then stirred at the same temperature for 22 hours. A crystal was collected from the resultant mixture through filtration and the crystal was washed with 5 ml of ethanol. The resultant crystal was dried to obtain 1.96 g of a diastereomer salt of (R)-isopropylpiperidin-3-yl carbamate and L-tartaric acid as a white crystal. Yield: 36.2%.

In the same manner as in Example 1, the crystal was analyzed by high-performance liquid chromatography. As a result, the optical purity of isopropylpiperidin-3-yl carbamate in the diastereomer salt was 96.8% ee (R-form).

### Example 3 to 5: Optical resolution of isopropylpiperidin-3-yl carbamate

The same operation as in Example 2 was conducted, except that the solvent described in Table 1 was used in place of the mixed solvent of methanol and 1-butanol (1/1 (volume/volume)) in Example 2. The results are shown in Table 1.

### Example 6: Optical resolution of ethylpiperidin-3-yl carbamate

The same operation as in Example 2 was conducted, except that 2.0g (11.6 mmol) of ethylpiperidin-3-yl carbamate was used in place of 3.0 g (16.1 mmol) of isopropylpiperidin-3-yl carbamate and methanol was used in place of the mixed solvent of methanol and 1-butanol (1/1 (volume/volume)) in Example 2, 1.15 g of a diastereomer salt of (R)-ethylpiperidin-3-yl carbamate and L-tartaric acid was obtained as a white crystal. Yield: 30.7%.
¹H-NMR (DMSO-d₆, 400MHz) δ ppm: 7.35 (1H, d, J=7Hz), 4.01-3.97 (4H, m), 3.65 (1H, br), 3.19 (1H, d-like), 3.10 (1H, d-like), 2.74 (1H, t-like), 2.63 (1H, t-like), 1.90-1.70 (2H, m), 1.64-1.61 (1H, m), 1.44-1.39 (1H, m), 1.16 (3H, d, J=7Hz) ¹³C-NMR (DMSO-d6, 400MHz) δ ppm: 174.6, 155.4, 71.9, 59.8, 46.6, 44.7, 42.6, 28.5, 20.7, 14.6

Using triethylamine, ethylpiperidin-3-yl carbamate was taken out from the diastereomer salt, derivatized by 3,5-dinitrobenzoyl chloride and then analyzed by high-performance liquid chromatography. As a result, the optical purity of ethylpiperidin-3-yl carbamate in the diastereomer salt was 94.3% ee (R-form).

### <Conditions for analysis of optical purity>

Column: CHIRALCEL AS-RH (4.6*150 mm, 5 µm)

Mobile phase: A = water, B = acetonitrile, A/B = 65/35 Flow rate: 1.0 ml/min

Detector: UV 254 nm

Retention time: S-form = 8.3 minutes, R-form = 18.2 minutes

### Example 7: Optical resolution of ethylpiperidin-3-yl carbamate

The same operation as in Example 6 was conducted, except that a mixed solvent of methanol and 1-butanol (1/1
(volume/volume)) was used in place of methanol in Example 6. The results are shown in Table 1.

### Example 8: Optical resolution of propylpiperidin-3-yl carbamate

The same operation as in Example 2 was conducted, except that 1.0 g (5.4 mmol) of propylpiperidin-3-yl carbamate was used in place of 3.0 g (16.1 mmol) of isopropylpiperidin-3-yl carbamate and ethanol was used in place of the mixed solvent of methanol and 1-butanol (1/1 (volume/volume)) in Example 2, 0.46 g of a diastereomer salt of (R)-propylpiperidin-3-yl carbamate and L-tartaric acid was obtained as a white crystal. Yield: 25.5%.
¹H-NMR (DMSO-d₆, 400MHz) δ ppm 7.34 (1H, d, J=7Hz), 3.97 (2H, s), 3.90 (2H, t, J=7Hz), 3.70-3.60 (1H, br), 3.19 (1H, d-like), 3.10 (1H, d-like), 2.73 (1H, t-like), 2.62 (1H, t-like), 1.85-1.70 (2H, m), 1.65-1.50 (3H, m), 1.48-1.31 (1H, m), 0.88
(3H, t, J=7Hz)
¹³C-NMR (DMSO-d6, 400MHz) δ ppm: 174.4, 155.5, 71.8, 65. 4, 46.7, 44.8, 42.7, 28.4, 22.0, 20.8, 10.3

Using triethylamine, propylpiperidin-3-yl carbamate was taken out from the diastereomer salt, derivatized by 3,5-dinitrobenzoyl chloride and then analyzed by high-performance liquid chromatography. As a result, the optical purity of propylpiperidin-3-yl carbamate in the diastereomer salt was 90.0% ee (R-form).

### <Conditions for analysis of optical purity>

Column: CHIRALCEL AS-RH (4.6*150 mm, 5 µm)

Mobile phase: A = water, B = acetonitrile, A/B = 65/35

Flow rate: 1.0 ml/min

Detector: UV 254 nm

Retention time: S-form = 12.5 minutes, R-form = 23.8 minutes

### Example 9: Optical resolution of isobutylpiperidin-3-yl carbamate

The same operation as in Example 2 was conducted, except that 2.0 g (10.0 mmol) of isobutylpiperidin-3-yl carbamate was used in place of 3.0 g (16.1 mmol) of isopropylpiperidin-3-yl carbamate and ethanol was used in place of the mixed solvent of methanol and 1-butanol (1/1 (volume/volume)) in Example 2, 0.91 g of a diastereomer salt of (R)-isobutylpiperidin-3-yl carbamate and L-tartaric acid was obtained as a white crystal. Yield: 26.0%.
¹H-NMR (DMSO-d₆, 400MHz) δ ppm: 7.34 (1H, d, J=7Hz), 3.95 (2H, s), 3.74 (2H, d, J=6Hz), 3.67-3.60 (1H, br), 3.19 (1H, d-like), 3.10 (1H, d-like), 2.73 (1H, t-like), 2.62 (1H, t-like), 1.86-1.70 (3H, m), 1.65-1.55 (1H, m), 1.48-1.35 (1H, m), 0.89
(6H, d, J=6Hz)
¹³C-NMR (DMSO-d6, 400MHz) δ ppm: 174.3, 155.5, 71.6, 69.8, 46.7, 44.8, 42.8, 28.4, 27.6, 20.9, 18.9

Using triethylamine, isobutylpiperidin-3-yl carbamate was taken out from the diastereomer salt, derivatized by 3,5-dinitrobenzoyl chloride and then analyzed by high-performance liquid chromatography. As a result, the optical purity of isobutylpiperidin-3-yl carbamate in the diastereomer salt was 86.4% ee (R-form).

### <Conditions for analysis of optical purity>

Column: CHIRALCEL AS-RH (4.6*150 mm, 5 µm)

Mobile phase: A = water, B = acetonitrile, A/B = 65/35 Flow rate: 1.0 ml/min

Detector: UV 254 nm

Retention time: S-form = 19.8 minutes, R-form = 37.7 minutes

### Examples 10 and 11: Optical resolution of isobutylpiperidin-3-yl carbamate

The same operation as in Example 8 was conducted, except that the solvent described in Table 1 was used in place of ethanol in Example 9. The results are shown in Table 1.

### Comparative Examples 1 to 4

The same operation as in Example 2 was conducted, except that 1.0 g (6.3 mmol) of methylpiperidin-3-yl carbamate was used in place of 3.0 g (16.1 mmol) of isopropylpiperidin-3-yl carbamate and the solvent described in Table 1 was used in place of the mixed solvent of methanol and 1-butanol (1/1 (volume/volume)) in Example 2, no crystal was precipitated in all Examples.

The above results are shown in Table 1.

**Table 1**

| | R | Solvent | Yield | Optical purity of crystal |
|---|---|---|---|---|
| Example 1 | Isopropyl group | Ethanol | 38.5% | 93.5%ee |
| Example 2 | | Mixed solvent 1* | 36.2% | 96.8%ee |
| Example 3 | | Mixed solvent 2* | 22.3% | 98.2%ee |
| Example 4 | | 2-propanol | 46.5% | 84.1%ee |
| Example 5 | | 1-butanol | 42.6% | 94.8%ee |
| Example 6 | Ethyl group | Methanol | 30.7% | 94.3%ee |
| Example 7 | | Mixed solvent 1* | 37.4% | 88.5%ee |
| Example 8 | Propyl group | Ethanol | 25.5% | 90.0%ere |
| Example 9 | Isobutyl group | Ethanol | 26.0% | 86.4%ere |
| Example 10 | | Mixed solvent 3* | 28.6% | 83.3%ee |
| Example 11 | | Mixed solvent 4* | 21.7% | 94.5%ere |
| Comparative Example 1 | Methyl group | Methanol | No crystal precipitation | |
| Comparative Example 2 | | Ethanol | | |
| Comparative Example 3 | | 2-propanol | | |
| Comparative Example 4 | | Mixed solvent 1* | | |

| | | | | |
|---|---|---|---|---|
| * Mixed solvent 1: methanol/1-butanol = 1/1 (volume/volume) Mixed solvent 2: methanol/1-butanol = 2/1 (volume/volume) Mixed solvent 3: methanol/1-butanol = 1/4 (volume/volume) Mixed solvent 4: methanol/1-butanol = 1/2 (volume/volume) | | | | |

### Comparative Example 5

The same operation as in Example 2 was conducted, except that 284 mg (2.0 mmol) of 3-acetylaminopiperidine was used in place of 3.0 g (16.1 mmol) of isopropylpiperidin-3-yl carbamate and ethanol was used in place of the mixed solvent of methanol and 1-butanol (1/1 (volume/volume)) in Example 2, no crystal was precipitated.

### Example 12: Production of (R)-isopropylpiperidin-3-yl carbamate

2.93 g (8.91 mmol, optical purity of 92.9% ee (R-form)) of a diastereomer salt obtained in the same manner as in Example 1 was mixed with 10 ml of water and 20 ml of ethyl acetate, and 1.94 g (18.29 mmol) of sodium carbonate was added while maintaining the resultant mixture at 20 to 25°C, followed by stirring, and an organic layer was obtained by a liquid separation treatment. The aqueous layer was extracted with 20 ml of ethyl acetate, and then the resultant organic layer and the organic layer obtained previously were combined. The resultant organic layer was dried over anhydrous sodium sulfate. Sodium sulfate was removed by filtration and the resultant solution was concentrated to obtain 1.58 g of (R)-isopropylpiperidin-3-yl carbamate as a white crystal. Yield: 97%.
¹H-NMR (DMSO-d₆, 400MHz) δ ppm: 6.85 (1H, d, J=7.8), 4.74-4.68 (1H, m), 3.30-3.15 (1H, m), 2.85 (1H, d-like, J=11.7Hz), 2.69 (1H, d-like, J=12.2Hz), 2.30 (1H, t-like, J=10.2Hz), 2.19 (1H, t-like, J=11.2Hz), 2.10-1.95 (1H, m), 1.80-1.68 (1H, m), 1.58-1.49 (1H, m), 1.35-1.18 (2H, m), 1.14 (6H, d, J=6.3Hz) ¹³C-NMR (DMSO-d₆, 400MHz) δ ppm: 155.0, 66.2, 51.6, 47.8, 45.6, 31.0, 25.2, 22.1

### Example 13: Production of (R)-ethylpiperidin-3-yl carbamate

3.0 g (9.31 mmol, optical purity of 93.4% ee (R-form)) of a diastereomer salt obtained in the same manner as in Example 6 was mixed with 10 ml of water and 20 ml of ethyl acetate, and 2.02 g (19.09 mmol) of sodium carbonate was added while maintaining the resultant mixture at 20 to 25°C, followed by stirring, and an organic layer was obtained by a liquid separation treatment. The aqueous layer was extracted with 20 ml of ethyl acetate, and then the resultant organic layer and the organic layer obtained previously were combined. The resultant organic layer was dried over anhydrous sodium sulfate. Sodium sulfate was removed by filtration and the resultant solution was concentrated to obtain 1.46 g of (R)-ethylpiperidin-3-yl carbamate as a white crystal. Yield: 91%.
¹H-NMR (DMSO-d₆, 400MHz) δ ppm: 6.93 (1H, d, J=8Hz), 3.97-3.92 (2H, m), 3.33-3.18 (1H, m), 2.86 (1H, d-like), 2.70 (1H, d-like), 2.31 (1H, t-like), 2.20 (1H, t-like), 1.78-1.68 (1H, m), 1.58-1.48 (1H, m), 1.38-1.20 (2H, m), 1.13 (3H, t, J=7Hz) ¹³C-NMR (DMSO-d₆, 400MHz) δ ppm: 155.4, 59.4, 51.3, 47.7, 45.5, 30.9, 25.0, 14.7

### Example 14: Production of (R)-propylpiperidin-3-yl carbamate

2.50 g (7.43 mmol, optical purity of 87.3% ee (R-form)) of a diastereomer salt obtained in the same manner as in Example 8 was mixed with 10 ml of water and 20 ml of ethyl acetate, and 1.61 g (15.23 mmol) of sodium carbonate was added while maintaining the resultant mixture at 20 to 25°C, followed by stirring, and an organic layer was obtained by a liquid separation treatment. The aqueous layer was extracted with 20 ml of ethyl acetate, and then the resultant organic layer and the organic layer obtained previously were combined. The resultant organic layer was dried over anhydrous sodium sulfate. Sodium sulfate was removed by filtration and the resultant solution was concentrated to obtain 1.31 g of (R)-propylpiperidin-3-yl carbamate as a white crystal. Yield: 95%.
¹H-NMR (DMSO-d₆, 400MHz) δ ppm: 6.95 (1H, d, J=8Hz), 3.87 (2H, t, J=7Hz), 3.28-3.26 (1H, m), 2.88 (1H, d-like), 2.72 (1H, d-like), 2.33 (1H, t-like), 2.23 (1H, t-like), 1.76-1.74 (1H, m), 1.59-1.50 (3H, m), 1.34-1.24 (2H, m), 0.88 (3H, t, J=7Hz) ¹³C-NMR (DMSO-d₆, 400MHz) δ ppm: 155.5, 65.0, 51.5, 47.8, 45.6, 31.0, 25.2, 22.1, 10.3

### Example 15: Production of (R)-isobutylpiperidin-3-yl carbamate

3.0 g (8.56 mmol, optical purity of 94.1% ee (R-form)) of a diastereomer salt obtained in the same manner as in Example 11 was mixed with 10 ml of water and 20 ml of ethyl acetate, and 1.86 g (17.55 mmol) of sodium carbonate was added while maintaining the resultant mixture at 20 to 25°C, followed by stirring, and an organic layer was obtained by a liquid separation treatment. The aqueous layer was extracted with 20 ml of ethyl acetate, and then the resultant organic layer and the organic layer obtained previously were combined. The resultant organic layer was dried over anhydrous sodium sulfate. Sodium sulfate was removed by filtration and the resultant solution was concentrated to obtain 1.67 g of (R)-isobutylpiperidin-3-yl carbamate as a white crystal. Yield: 97%.
¹H-NMR (DMSO-d₆, 400MHz) δ ppm: 6.94 (1H, d, J=8Hz), 3.70 (2H, d, J=6Hz), 3.28-3.22 (1H, m), 2.87 (1H, d-like), 2.71 (1H, d-like), 2.32 (1H, t-like), 2.22 (1H, t-like), 1.85-1.74 (2H, m), 1.57-1.53 (1H, m), 1.34-1.24 (2H, m), 0.87 (6H, d, J=7Hz) ¹³C-NMR (DMSO-d₆, 400MHz) δ ppm: 155.5, 69.5, 51.6, 47.9, 45.6, 31.0, 27.6, 25.2, 18.9

### Example 16: Production of (R)-3-aminopiperidine

3.0 g (8.92 mmol) of the diastereomer salt obtained in Example 1 was mixed with 4.65 g (44.6 mmol) of 35 wt% hydrochloric acid and the resultant mixture was stirred at 90°C for 10 hours. After the completion of the reaction, water was distilled off by concentrating the resultant mixture under reduced pressure. Furthermore, an operation of adding 10 ml of 1-butanol and concentrating under reduced pressure was conducted twice. To the resultant oily product, 10 ml of 1-butanol was added, followed by stirring at room temperature to precipitate a crystal. The crystal was collected by filtration and then washed with 5 ml of 1-butanol. The resultant crystal was dried to obtain 1.25 g of a hydrochloride of (R)-3-aminopiperidine as a white crystal. Yield: 81%.

Using triethylamine, 3-aminopiperidine was taken out from the hydrochloride, derivatized by 3,5-dinitrobenzoylchloride and then analyzed by high-performance liquid chromatography. As a result, the optical purity of 3-aminopiperidine in the hydrochloride was 95.4% ee (R-form).

### <Conditions for analysis of optical purity>

Column: CHIRALCEL AS-RH (4.6*150 mm, 5 µm)

Mobile phase: A = water, B = acetonitrile, A/B = 65/35 Flow rate: 1.0 ml/min

Detector: UV 254 nm

Retention time: S-form = 21.3 minutes, R-form = 23.4 minutes

### Industrial Applicability

The optically active carbamate compound (3) and optically active 3-aminopiperidine are useful as an intermediate for synthesis of a remedy for diabetes mellitus (see International Publication No. WO 2005/085246 and International Publication No. WO 2006/112331), and the present invention can be industrially applied as a method of producing such an intermediate.

## Claims

1. A method for optical resolution of an alkylpiperidin-3-yl carbamate, the method comprising bringing an RS mixture of an alkylpiperidin-3-yl carbamate represented by the formula (1): 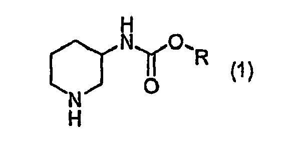 wherein R represents an alkyl group having 2 to 4 carbon atoms, into contact with an optically active tartaric acid in the presence of a solvent.

2. The method for optical resolution according to claim 1, wherein the optically active tartaric acid is L-tartaric acid.

3. A method for producing an optically active alkylpiperidin-3-yl carbamate represented by the formula (3): 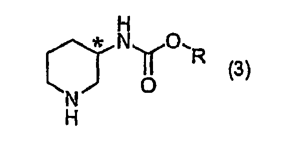 or a salt thereof,
wherein R and * are as defined below, the method comprising bringing an RS mixture of an alkylpiperidin-3-yl carbamate
represented by the formula (1): 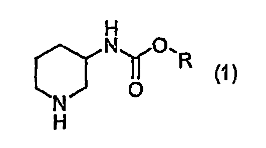 wherein R represents an alkyl group having 2 to 4 carbon atoms, into contact with an optically active tartaric acid in the presence of a solvent to crystallize a diastereomer salt of an optically active alkylpiperidin-3-yl carbamate represented by the formula (2): 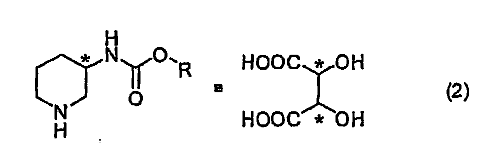 wherein R is as defined above and * represents that the carbon atom is an optically active center, and an optically active tartaric acid; and then reacting the diastereomer salt with an acid or a base at 0°C or higher and lower than 60°C.

4. The production method according to claim 3, wherein the optically active tartaric acid is L-tartaric acid and the resultant optically active alkylpiperidin-3-yl carbamate represented by the formula (3) or a salt thereof is the R-form.

5. A method for producing an optically active 3-aminopiperidine or a salt thereof, the method comprising bringing an RS mixture of an alkylpiperidin-3-yl carbamate represented by the formula (1): 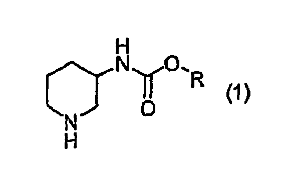 wherein R represents an alkyl group having 2 to 4 carbon atoms, into contact with an optically active tartaric acid in the presence of a solvent to crystallize a diastereomer salt of an optically active alkylpiperidin-3-yl carbamate represented by the formula (2): 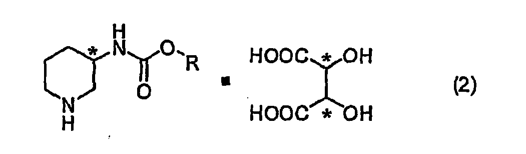 wherein R is as defined above and * represents that the carbon atom is an optically active center, and an optically active tartaric acid; and then reacting the diastereomer salt with an acid or a base at 60°C or higher and 150°C or lower.

6. The production method according to claim 5, wherein the optically active tartaric acid is L-tartaric acid and the resultant optically active 3-aminopiperidine or a salt thereof is the R-form.

7. The production method according to any one of claims 3 to 6, wherein R in the formula (1) is an ethyl group, and the solvent is methanol or a mixed solvent of methanol and an alcohol having 2 to 4 carbon atoms.

8. The production method according to any one of claims 3 to 6, wherein R in the formula (1) is an ethyl group, and the solvent is methanol or a mixed solvent of methanol and 1-butanol.

9. The production method according to any one of claims 3 to 6, wherein R in the formula (1) is an alkyl group having 3 or 4 carbon atoms, and the solvent is at least one alcohol solvent selected from alcohols having 1 to 4 carbon atoms.

10. The production method according to any one of claims 3 to 6, wherein R in the formula (1) is an alkyl group having 3 or 4 carbon atoms, and the solvent is a mixed solvent of methanol and an alcohol having 2 to 4 carbon atoms, or an alcohol having 2 to 4 carbon atoms.

11. The production method according to any one of claims 3 to 6, wherein R in the formula (1) is a propyl group, and the solvent is ethanol.

12. The production method according to any one of claims 3 to 6, wherein R in the formula (1) is an isopropyl group, and the solvent is ethanol, 2-propanol, 1-butanol, or a mixed
solvent of methanol and 1-butanol.

13. The production method according to any one of claims 3 to 6, wherein R in the formula (1) is an isobutyl group, and the solvent is a mixed solvent of methanol and 1-butanol, or ethanol.

14. A diastereomer salt of an optically active alkylpiperidin-3-yl carbamate represented by the formula (2): 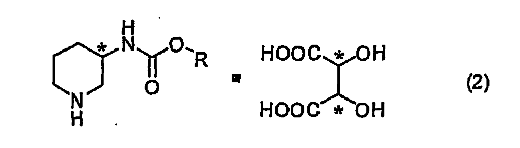 wherein R represents an alkyl group having 2 to 4 carbon atoms and * represents that the carbon atom is an optically active center, and an optically active tartaric acid.

15. A diastereomer salt of an (R)-alkylpiperidin-3-yl carbamate represented by the formula (2a): 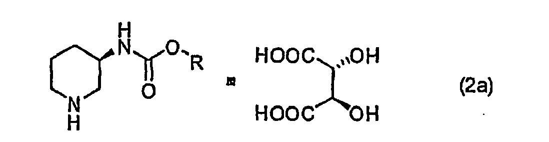 wherein R represents an alkyl group having 2 to 4 carbon atoms, and L-tartaric acid.

16. An optically active alkylpiperidin-3-yl carbamate represented by the formula (3): 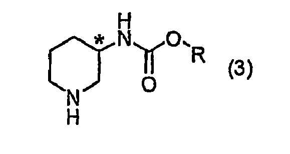 wherein R represents an alkyl group having 2 to 4 carbon atoms and * represents that the carbon atom is an optically active center.

17. An (R)-alkylpiperidin-3-yl carbamate represented by the formula (3a): 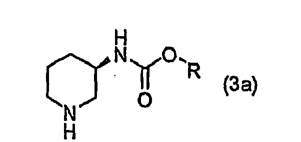 wherein R represents an alkyl group having 2 to 4 carbon atoms.

18. An alkylpiperidin-3-yl carbamate represented by the formula (1): 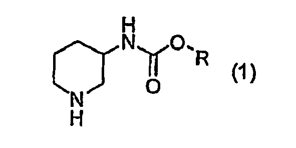 wherein R represents a propyl group or an isopropyl group.
